# EUROPÄISCHE PATENTSCHRIFT

(11) **EP 0 930 356 B1**
(45) Veröffentlichungstag und Bekanntmachung des Hinweises auf die Patenterteilung: **05.11.2003**
(21) Anmeldenummer: 99100169.4
(22) Anmeldetag: 07.01.1999
(51) Int. Cl.: C11D 3/20, A61K 7/50, C11D 11/00

(54) **Verfahren zur Herstellung von fliessfähigen Perlglanz- und Trübungsmittelkonzentraten**
Process for making flowable pearlescent and opacicying concentrates
Procédé pour la préparation de concentrés nacrés et opacifiant s'écoulant librement

(30) Priorität: 15.01.1998 DE 19801231
(43) Veröffentlichungstag der Anmeldung: 21.07.1999
(73) Patentinhaber: Cognis Deutschland GmbH & Co. KG, 40589 Düsseldorf (DE)
(72) Erfinder: Wadle, Armin Dr., 40699 Erkrath (DE); Lehen-Ferrenbach, Catherine, 77100 Meaux (FR); Morand, Corinne, 77100 Meaux (FR)

(56) Entgegenhaltungen:
- EP-A- 0 568 848
- US-A- 4 486 334
- DATABASE WPI Section Ch, Week 8321 Derwent Publications Ltd., London, GB; Class A25, AN 83-49785K XP002107661 & JP 58 038798 A (UNILEVER NV) , 7. März 1983
- DATABASE WPI Section Ch, Week 9431 Derwent Publications Ltd., London, GB; Class D21, AN 94-251837 XP002107662 & JP 06 182173 A (KAO CORP), 5. Juli 1994
- DATABASE WPI Section Ch, Week 8828 Derwent Publications Ltd., London, GB; Class D21, AN 88-194752 XP002107664 & JP 63 132973 A (LION CORP), 4. Juni 1988
- DATABASE WPI Section Ch, Week 8829 Derwent Publications Ltd., London, GB; Class D21, AN 88-202118 XP002107665 & JP 63 139996 A (LION CORP) , 11. Juni 1988

## Beschreibung

### Gebiet der Erfindung

Die Erfindung betrifft ein Verfahren zur Herstellung von fließfähigen Perlglanz- und Trübungsmittelkonzentraten, bei dem man einen wäßrigen Wachskörperpremix versprüht.

### Stand der Technik

Der weich schimmernde Glanz von Perlen hat auf den Menschen schon seit Jahrtausenden eine besondere Faszination ausgeübt. Es ist daher kein Wunder, daß die Hersteller von kosmetischen Zubereitungen versuchen, ihren Produkten ein attraktives, wertvolles und gehaltvolles Erscheinungsbild zu verleihen. Der erste seit dem Mittelalter in der Kosmetik eingesetzte Perlglanz war eine perlglänzende Paste aus natürlichen Fischschuppen. Zu Anfang dieses Jahrhunderts entdeckte man, daß Wismutoxidchloride ebenfalls in der Lage sind, Perlglanz zu erzeugen. Für die moderne Kosmetik sind hingegen Perlglanzwachse, insbesondere vom Typ der Glycolmono- und -difettsäureester von Bedeutung, die überwiegend zur Erzeugung von Perlglanz in Haarshampoos und Duschgelen eingesetzt werden. Eine Übersicht zu modernen, perlglänzenden Formulierungen findet sich von A.Ansmann und R.Kawa in **Parf.Kosm. 75, 578 (1994).** Neben perlglänzenden Formulierungen werden im Markt häufig auch Formulierungen gefunden, die eine intensive Weißtrübung aufweisen. Zu ihrer Herstellung werden Trübungsmittel eingesetzt, bei denen es sich ebenfalls um Dispersionen von Wachskörpern handelt.

Der Stand der Technik kennt eine Vielzahl von Formulierungen, die oberflächenaktiven Mitteln den gewünschten Perlglanz bzw. Weißtrübung verleihen. So sind beispielsweise aus den beiden Deutschen Patentanmeldungen **DE-A1 3843572** und **DE-A1 4103551** (Henkel) Perlglanzkonzentrate in Form fließfähiger wäßriger Dispersionen bekannt, die 15 bis 40 Gew.-% perlglänzender Komponenten, 5 bis 55 Gew.-% Emul-gatoren und 0,1 bis 5 bzw. 15 bis 40 Gew.-% Polyole enthalten. Bei den Perlglanzwachsen handelt es sich um acylierte Polyalkylenglycole, Monoalkanolamide, lineare, gesättigte Fettsäuren oder Ketosulfone. In den beiden Europäischen Patentschriften **EP-B1 0181773** und **EP-B1** **0285389** (Procter & Gamble) werden Shampoozusammensetzungen vorgeschlagen, die Tenside, nicht-flüchtige Silicone und Perlglanzwachse enthalten. Gegenstand der Europäischen Patentanmeldung **EP-A2 0205922** (Henkel) sind fließfähige Perlglanzkonzentrate, die 5 bis 15 Gew.-% acylierte Polyglycole, 1 bis 6 Gew.-% Fettsäuremonoethanolamide und 1 bis 5 Gew.-% nichtionische Emulgatoren enthalten. Gemäß der Lehre der Europäischen Patentschrift **EP-B1 0569843** (Hoechst) lassen sich nichtionische, fließfähige Perlglanzdispersionen auch erhalten, indem man Mischungen von 5 bis 30 Gew.-% acylierten Polyglycolen und 0,1 bis 20 Gew.-% ausgewählten nichtionischen Tensiden herstellt. Aus der Europäischen Patentanmeldung **EP-A2 0581193** (Hoechst) sind ferner fließfähige, konservierungsmittelfreie Perlglanzdispersionen bekannt, die acylierte Polyglycolether, Betaine, Aniontenside und Glycerin enthalten. Schließlich wird in der Europäischen Patentanmeldung **EP-A1 0684302** (Th.Goldschmidt) die Verwendung von Polyglycerinestem als Kristallisationshilfsmittel für die Herstellung von Perlglanzkonzentraten vorgeschlagen. Trübungmittel mit einem Gehalt an Alkyloligoglucosiden und Monoglyceriden sind aus der Deutschen Patentschrift **DE-C2 19511572** (Henkel) bekannt.

Zur Herstellung der perlglänzenden oder weißgetrübten Endformulierungen werden üblicherweise konzentrierte Wachskörperdispersionen eingesetzt, welche man gewinnt, indem man eine wäßrige Dispergatorlösung über den Schmelzpunkt des Wachskörpers erwärmt, diesen einrührt und die Zubereitung dann definiert abkühlen läßt. Im Verlauf des Abkühlens bilden die Wachskörper Kristalle, an denen sich das Licht bricht. Die Brillanz des Perlglanz bzw. der Weißtrübung wird dabei maßgeblich durch Form und Größe der Kristalle bestimmt, welche ihrerseits im wesentlichen durch die Abkühlgeschwindigkeit beeinflußt werden können. Dabei gilt: Je kleiner die Kristalle um so länger der Abkühlvorgang. Insbesondere dann, wenn besonders feine Kristalle erhalten werden sollen, ist die übliche Herstellung von konzentrierten Wachskörperdispersionen energie- und zeitaufwendig, was sich direkt auf die Herstellkosten auswirkt.

Demzufolge besteht Interesse an einem Verfahren, welches die Herstellung von fließfähigen, lagerstabilen Perlglanz- bzw. Trübungsmittelkonzentraten gestattet und dabei mit einem verminderten technischen Aufwand, einer geringeren Energiemenge und einem reduzierten Zeitbedarf auskommt. Gleichzeitig sollten Teilchenform und Teilchendurchmesser durch die Prozeßparameter leicht beeinflußbar sein. Die Aufgabe der Erfindung hat darin bestanden, ein solches Verfahren zur Verfügung zu stellen.

### Beschreibung der Erfindung

Gegenstand der Erfindung ist ein Verfahren zur Herstellung von fließfähigen Perlglanz- und Trübungsmittelkonzentraten, bei dem man in einem ersten Reaktor einen wäßrigen Wachskörperpremix herstellt und diesen in einen zweiten mit Wasser gefüllten Reaktor versprüht.

Gegenüber den bekannten Verfahren zur Herstellung von Perlglanz- oder Trübungsmittelkonzentraten zeichnet sich die Erfindung durch einen verminderten Zeit- und Energiebedarf aus, zudem läßt sich über die Prozeßparameter (Homogenisierzeit, Düseneinstellung, Temperatur) die mittlere Kristallgröße im Bereich von 0,5 bis 50 µm exakt einstellen. In Abhängigkeit der ausgewählten Wachskörper bzw. Wachskörpermischungen werden fließfähige Perlglanz- oder Trübungsmittelkonzentrate erhalten, die sich durch eine ausgezeichnete Lagerstabilität auszeichnen.

### Wachskörper

Als Wachskörper kommen beispielsweise in Frage: Alkylenglycolester; Fettsäurealkanolamide; Partialglyceride; Ester von mehrwertigen, gegebenenfalls hydroxysubstituierte Carbonsäuren mit Fettalkoholen mit 6 bis 22 Kohlenstoffatomen; Fettstoffe, wie beispielsweise Fettalkohole, Fettketone, Fettaldehyde, Fettether und Fettcarbonate, die in Summe mindestens 24 Kohlenstoffatome aufweisen; Alkandiole mit 10 bis 18 Kohlenstoffatomen, gegebenenfalls hydroxyfunktionalisierte Fettsäuren mit 18 bis 24 Kohlenstoffatomen, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.
- **Alkylenglycolester.** Bei den Alkylenglycolestem handelt es sich üblicherweise um Mono- und/oder Diester von Alkylenglycolen, die der Formel **(I)** folgen,

   **R**^{**1**}**CO(OA)**_{**n**}**OR**^{**2**} **(I)**

   in der R¹CO für einen linearen oder verzweigten, gesättigten oder ungesättigten Acylrest mit 6 bis 22 Kohlenstoffatomen, R² für Wasserstoff oder R¹CO und A für einen linearen oder verzweigten Alkylenrest mit 2 bis 4 Kohlenstoffatomen und n für Zahlen von 1 bis 5 steht. Typische Beispiele sind Mono- und/oder Diester von Ethylenglycol, Propylenglycol, Diethylenglycol, Dipropylenglycol, Triethylenglycol oder Tetraethylenglycol mit Fettsäuren mit 6 bis 22, vorzugsweise 12 bis 18 Kohlenstoffatomen als da sind: Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Besonders bevorzugt ist der Einsatz von Ethylenglycolmono- und/oder -distearat.
- **Fettsäurealkanolamide.** Fettsäurealkanolamide, die als Wachskörper in Frage kommen, folgen der Formel **(II),**

   **R**^{**3**}**CO-NR**^{**4**}**-B-OH** **(II)**

   in der R³CO für einen linearen oder verzweigten, gesättigten oder ungesättigten Acylrest mit 6 bis 22 Kohlenstoffatomen, R⁴ für Wasserstoff oder einen gegebenenfalls hydroxysubstituierten Alkylrest mit 1 bis 4 Kohlenstoffatomen und B für eine lineare oder verzweigte Alkylengruppe mit 1 bis 4 Kohlenstoffatomen steht. Typische Beispiele sind Kondensationsprodukte von Ethanolamin, Methylethanolamin, Diethanolamin, Propanolamin, Methylpropanolamin und Dipropanolamin sowie deren Mischungen mit Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Besonders bevorzugt ist der Einsatz von Stearinsäureethanolamid.
- **Partialglyceride.** Partialglyceride, die über Perlglanzeigenschaften verfügen, stellen Monound/oder Diester des Glycerins mit Fettsäuren, nämlich beispielsweise Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen dar. Sie folgen der Formel (III), 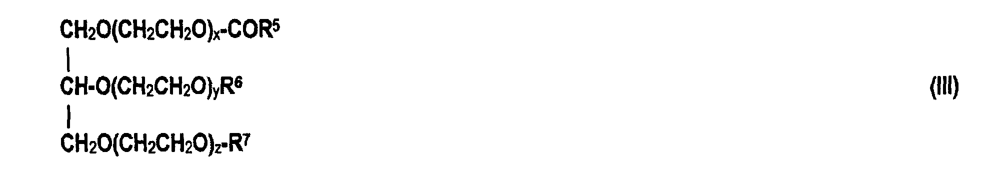 in der R⁵CO für einen linearen oder verzweigten Acylrest mit 6 bis 22 Kohlenstoffatomen, R⁶ und R⁷ unabhängig voneinander für Wasserstoff oder R⁵CO, x, y und z in Summe für 0 oder für Zahlen von 1 bis 30 und X für ein Alkali- oder Erdalkalimetall mit der Maßgabe steht, daß mindestens einer der beiden Reste R⁶ und R⁷ Wasserstoff darstellt. Typische Beispiele sind Laurinsäuremonoglycerid, Laurinsäurediglycerid, Kokosfettsäuremonoglycerid, Kokosfettsäuretriglycerid, Palmitinsäuremonoglycerid, Palmitinsäuretriglycerid, Stearinsäuremonoglycerid, Stearinsäurediglycerid, Isostearinsäuremonoglycerid, Isostearinsäurediglycerid, Ölsäuremonoglycerid, Ölsäurediglycerid, Talgfettsäuremonoglycerid, Talgfettsäurediglycerid, Behensäuremonoglycerid, Behensäurediglycerid, Erucasäuremonoglycerid, Erucasäurediglycerid sowie deren technische Gemische, die untergeordnet aus dem Herstellungsprozeß noch geringe Mengen an Triglycerid enthalten können.
- **Mehrwertige Carbonsäure- und Hydroxycarbonsäureester.** Als Wachskörper kommen weiterhin Ester von mehrwertigen, gegebenenfalls hydroxysubstituierten Carbonsäuren mit Fettalkoholen mit 6 bis 22 Kohlenstoffatomen in Frage. Als Säurekomponente dieser Ester kommen beispielsweise Malonsäure, Maleinsäure, Fumarsäure, Adipinsäure, Sebacinsäure, Azelainsäure, Dodecandisäure, Phthalsäure, Isophthalsäure und insbesondere Bernsteinsäure sowie Äpfelsäure, Citronensäure und insbesondere Weinsäure und deren Mischungen in Betracht. Die Fettalkohole enthalten 6 bis 22, vorzugsweise 12 bis 18 und insbesondere 16 bis 18 Kohlenstoffatome in der Alkylkette. Typische Beispiele sind Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen. Die Ester können als Voll- oder Partialester vorliegen, vorzugsweise werden Mono- und vor allem Diester der Carbon- bzw. Hydroxycarbonsäuren eingesetzt. Typische Beispiele sind Bemsteinsäuremono- und -dilaurylester, Bemsteinsäuremono- und -dicetearlyester, Bernsteinsäuremono- und -distearylester, Weinsäuremono- und -dilaurylester, Weinsäuremono- und -dikokosalkylester, Weinsäuremono- und -dicetearylester, Citronen-säuremono-, -di- und -trilaurylester, Citronensäuremono-, -di- und -trikokosalkylester sowie Citro-nensäuremono-, -di- und -tricetearylester.
- **Fettalkohole.** Als weitere Gruppe von Wachskörpern können langkettige Fettalkohole eingesetzt werden, die der Formel (IV) folgen,

   **R**^{**8**}**OH** **(IV)**

   in der R⁸ für einen linearen Alkylrest mit 24 bis 48, vorzugsweise 32 bis 36 Kohlenstoffatomen steht. Bei den genannten Stoffen handelt es sich in der Regel um Oxidationsprodukte langkettiger Paraffine.
- **Fettketone.** Fettketone, die als Wachskröper in Betracht kommen, folgen vorzugsweise der Formel **(V),**

   **R**^{**8**}**-CO-R**^{**9**} **(V)**

   in der R⁸ und R⁹ unabhängig voneinander für Alkyl- und/oder Alkenylreste mit 1 bis 22 Kohlenstoffatomen stehen, mit der Maßgabe, daß sie in Summe mindestens 24 und vorzugsweise 32 bis 48 Kohlenstoffatome aufweisen. Die Ketone können nach Verfahren des Stands der Technik hergestellt werden, beispielsweise durch Pyrolyse der entsprechenden Fettsäure-Magnesiumsalze. Die Ketone können symmetrisch oder unsymmetrisch aufgebaut sein, vorzugsweise unterscheiden sich die beiden Reste R⁸ und R⁹ aber nur um ein Kohlenstoffatom und leiten sich von Fettsäuren mit 16 bis 22 Kohlenstoffatomen ab. Dabei zeichnet sich Stearon durch besonders vorteilhafte Perlglanzeigenschaften aus.
- **Fettaldehyde.** Als Wachskörper geeignete Fettaldehyde entsprechen der Formel **(VI)**,

   **R**^{**10**}**COH** **(VI)**

   in der R¹⁰CO für einen linearen oder verzweigten Acylrest mit 24 bis 48, vorzugsweise 28 bis 32 Kohlenstoffatomen steht.
- **Fettether.** Als Wachskörper kommen ferner Fettether der Formel (VII) in Frage,

   **R**^{**11**}**-O-R**^{**12**} **(VII)**

   in der R¹¹ und R¹² unabhängig voneinander für Alkyl- und/oder Alkenylreste mit 1 bis 22 Kohlenstoffatomen stehen, mit der Maßgabe, daß sie in Summe mindestens 24 und vorzugsweise 32 bis 48 Kohlenstoffatome aufweisen. Fettether der genannten Art werden üblicherweise durch saure Kondensation der entsprechenden Fettalkohole hergestellt. Fettether mit besonders vorteilhaften Perlglanzeigenschaften werden durch Kondensation von Fettalkoholen mit 16 bis 22 Kohlenstoffatomen, wie beispielsweise Cetylalkohol, Cetearylalkohol, Stearylalkohol, Isostearylalkohol, Oleylalkohol, Behenylalkohol und/oder Erucylalkohol erhalten.
- **Fettcarbonate.** Als Wachskörper kommen weiterhin Fettcarbonate der Formel (VIII) in Betracht,

   **R**^{**13**}**O-CO-OR**^{**14**} **(VIII)**

   in der R¹³ und R¹⁴ unabhängig voneinander für Alkyl- und/oder Alkenylreste mit 1 bis 22 Kohlenstoffatomen stehen, mit der Maßgabe, daß sie in Summe mindestens 24 und vorzugsweise 32 bis 48 Kohlenstoffatome aufweisen. Die Stoffe werden erhalten, indem man beispielsweise Dimethyloder Diethylcarbonat mit den entsprechenden Fettalkoholen in an sich bekannter Weise umestert. Demzufolge können die Fettcarbonate symmetrisch oder unsymmetrisch aufgebaut sein. Vorzugsweise werden jedoch Carbonate eingesetzt, in denen R¹³ und R¹⁴ gleich sind und für Alkylreste mit 16 bis 22 Kohlenstoffatomen stehen. Besonders bevorzugt sind Umesterungsprodukte von Dimethyl- bzw. Diethylcarbonat mit Cetylalkohol, Cetearylalkohol, Stearylalkohol, Isostearylalkohol, Oleylalkohol, Behenylalkohol und/oder Erucylalkohol in Form ihrer Mono- und Diester bzw. deren technischen Mischungen.
- **Fettsäuren.** Als Wachskörper sind weiterhin Fettsäuren geeignet, die 18 bis 24 Kohlenstoffatome aufweisen und dabei hydroxyfunktionalisiert sein können. Typische Beispiele sind Stearinsäure, Hydroxystearinsäure und insbesondere Behensäure.
- **Epoxidringöffnungsprodukte.** Bei den Ringöffnungsprodukten handelt es sich um bekannte Stoffe, die üblicherweise durch säurekatalysierte Umsetzung von endständigen oder innenständigen Olefinepoxiden mit aliphatischen Alkoholen hergestellt werden. Die Reaktionsprodukte folgen vorzugsweise der Formel (IX), 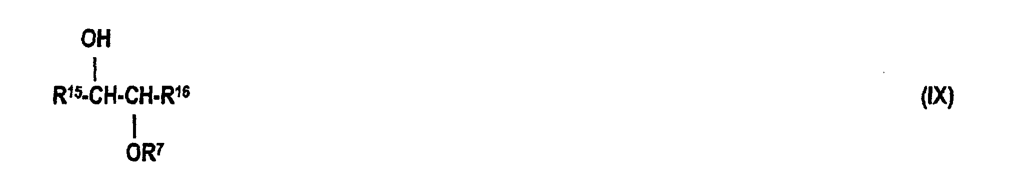 in der R¹⁵ und R¹⁶ für Wasserstoff oder einen Alkylrest mit 10 bis 20 Kohlenstoffatomen steht, mit der Maßgabe, daß die Summe der Kohlenstoffatome von R¹⁵ und R¹⁶ im Bereich von 10 bis 20 liegt und R¹⁷ für einen Alkyl- und/oder Alkenylrest mit 12 bis 22 Kohlenstoffatomen und/oder den Rest eines Polyols mit 2 bis 15 Kohlenstoffatomen und 2 bis 10 Hydroxylgruppen steht. Typische Beispiele sind Ringöffnungsprodukte von α-Dodecenepoxid, α-Hexadecenepoxid, α-Octadecenepoxid, α-Eicosenepoxid, α-Docosenepoxid, i-Dodecenepoxid, i-Hexadecenepoxid, i-Octadecenepoxid, i-Eicosenepoxid und/oder i-Docosenepoxid mit Laurylalkohol, Kokosfettalkohol, Myristylalkohol, Cetylalkohol, Cetearylalkohol, Stearylalkohol, Isostearylalkohol, Oleylalkohol, Elaidylalkohol, Petroselinylalkohol, Linolylalkohol, Linolenylalkohol, Behenylalkohol und/oder Erucylalkohol. Vorzugsweise werden Ringöffnungsprodukte von Hexa- und/oder Octadecenepoxiden mit Fettalkoholen mit 16 bis 18 Kohlenstoffatomen eingesetzt. Werden anstelle der Fettalkohole Polyole für die Ringöffnung eingesetzt, so handelt es sich beispielsweise um folgende Stoffe: Glycerin; Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton; technische Oligoglyceringemische mit einem Eigenkondensationsgrad von 1,5 bis 10 wie etwa technische Diglyceringemische mit einem Diglyceringehalt von 40 bis 50 Gew.-%; Methyolverbindungen, wie insbesondere Trimethylolethan, Trimethylolpropan, Trimethylolbutan, Pentaerythrit und Dipentaerythrit; Niedrigalkylglucoside, insbesondere solche mit 1 bis 8 Kohlenstoffen im Alkylrest, wie beispielsweise Methyl- und Butylglucosid; Zuckeralkohole mit 5 bis 12 Kohlenstoffatomen, wie beispielsweise Sorbit oder Mannit, Zucker mit 5 bis 12 Kohlenstoffatomen, wie beispielsweise Glucose oder Saccharose; Aminozucker, wie beispielsweise Glucamin.

### Emulgatoren

Die Wachskörper können direkt mit Wasser zu einem Premix verarbeitet werden. In einer bevorzugten Ausführungsform der Erfindung werden jedoch anionische, nichtionische, kationische, amphotere und/oder zwitterionische Emulgatoren mitverwendet, wobei die Emulgatormenge üblicherweise im Bereich von 0,5 bis 30, vorzugsweise 1 bis 20 und insbesondere 5 bis 10 Gew.-% - bezogen auf den Premix - liegt.

Typische Beispiele für anionische Emulgatoren sind Alkylethersulfate und/oder Monoglycerid(ether)-sulfate. Als nichtionische Emulgatoren kommen in Frage:
(b1) Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
(b2) C_{12/18}-Fettsäuremono- und -diester von Anlagerungsprodukten von 1 bis 30 Mol Ethylenoxid an Glycerin;
(b3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
(b4) Alkylmono- und -oligoglycoside mit 8 bis 22 Kohlenstoffatomen im Alkylrest und deren ethoxylierte Analoga;
(b5) Anlagerungsprodukte von 15 bis 60 Mol Ethylenoxid an Ricinusöl und/oder gehärtetes Ricinusöl;
(b6) Polyol- und insbesondere Polyglycerinester, wie z.B. Polyglycerinpolyricinoleat, Polyglycerinpoly-12-hydroxystearat oder Polyglycerindimerat. Ebenfalls geeignet sind Gemische von Verbindungen aus mehreren dieser Substanzklassen;
(b7) Anlagerungsprodukte von 2 bis 15 Mol Ethylenoxid an Ricinusöl und/oder gehärtetes Ricinusöl;
(b8) Partialester auf Basis linearer, verzweigter, ungesättigter bzw. gesättigter C_{6/22}-Fettsäuren, Ricinolsäure sowie 12-Hydroxystearinsäure und Glycerin, Polyglycerin, Pentaerythrit, Dipentaerythrit, Zuckeralkohole (z.B. Sorbit), Alkylglucoside (z.B. Methylglucosid, Butylglucosid, Laurylglucosid) sowie Polyglucoside (z.B. Cellulose);
(b9) Mono-, Di- und Trialkylphosphate sowie Mono-, Di- und/oder Tri-PEG-alkylphosphate;
(b10) Wollwachsalkohole und deren Ethylenoxidanlagerungsprodukte;
(b11) Polysiloxan-Polyalkyl-Polyether-Copolymere bzw. entsprechende Derivate;
(b12) Mischester aus Pentaerythrit, Fettsäuren, Citronensäure und Fettalkohol gemäß **DE-PS 1165574** und/oder Mischester von Fettsäuren mit 6 bis 22 Kohlenstoffatomen, Methylglucose und Polyolen, vorzugsweise Glycerin sowie
(b13) Polyalkylenglycole.

Die Anlagerungsprodukte von Ethylenoxid undloder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE-PS 20 24 051** als Rückfettungsmittel für kosmetische Zubereitungen bekannt.

C_{8/18}-Alkylmono- und -oligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Neben den ampholytischen kommen auch quartäre Emulgatoren in Betracht, wobei solche vom Typ der Esterquats, vorzugsweise methylquatemierte Difettsäuretriethanolaminester-Salze, besonders bevorzugt sind.

### Premix-Herstellung

Zur Herstellung des Premix wird im ersten Reaktor Wasser bzw. eine wäßrige Emulgatorlösung vorgelegt, auf 60 bis 100, vorzugsweise 70 bis 90°C erhitzt und die Wachskörper eingetragen, so daß sich eine Wachskörperkonzentration im Bereich von 5 bis 50, vorzugsweise 10 bis 40 Gew.-% einstellt. Die Mischung wird unter starker Scherung gerührt, wobei mit zunehmender Homogenisierzeit die mittlere Kristallgröße zu kleineren Teilchendurchmessern verschoben wird.

### Versprühung

Die Versprühung des Premix kann in an sich bekannter Weise durchgeführt werden, d.h. der Premix wird aus dem ersten Reaktor in eine Sprühvorrichtung gepumpt, mit deren Hilfe die Wachskörperdispersion auf die Oberfläche eines mit Wasser gefüllten zweiten Reaktors sprüht wird. Anstelle von Wasser kann der zweite Reaktor auch eine wäßrige Lösung anionischer, nichtionischer, kationischer, amphoterer und/oder zwitterionischer Tenside enthalten. Dies ist insbesondere dann sinnvoll, wenn der Premix selbst keine Emulgatoren enthält. Vorzugsweise weist das Wasser bzw. die wäßrige Tensidlösung im zweiten Reaktor eine Temperatur im Bereich von 10 bis 30°C auf, so daß die heißen Premixtröpfchen beim Auftreffen auf den Oberfläche spontan abgekühlt werden und auskristallisieren.

### Tenside

Werden im zweiten Reaktor anstelle vom Wasser wäßrige Tensidlösungen vorgelegt, so können diese einen Feststoffgehalt von 10 bis 50, vorzugsweise von 20 bis 40 Gew.-% aufweisen. Typische Beispiele für **anionische Tenside** sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Hydroxymischethersulfate, Monoglycerid(ether)sulfate, Fettsäureethersulfate, Fettsäureamid(ether)sulfate, Mono- und Dialkyl-sulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für **nichtionische Tenside** sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für **kationische Tenside** sind quartäre Ammoniumverbindungen und Esterquats, insbesondere quaternierte Fettsäuretrialkanolaminestersalze. Typische Beispiele für **amphotere bzw. zwitterionische Tenside** sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten beispielsweise **J.Falbe (ed.), "Surfactants in Consumer Products", Springer Verlag, Berlin, 1987, S. 54-124** oder **J.Falbe (ed.), "Katalysatoren, Tenside und Mineralöladditive", Thieme Verlag, Stuttgart, 1978, S. 123-217** verwiesen.

### Gewerbliche Anwendbarkeit

Die nach dem erfindungsgemäßen Verfahren erhältlichen Perlglanz- bzw. Trübungsmittelkonzentrate dienen als Rohstoffe zur Herstellung von oberflächenaktiven Mitteln, wie insbesondere Haarshampoos und Geschirrspülmitteln, denen sie entweder einen brillanten Perlglanz oder eine intensive Weißtrübung verleihen. Bezogen auf die Endformulierungen können die Konzentrate in Mengen von 0,5 bis 5 und vorzugsweise 1 bis 2 Gew.-% eingesetzt werden.

### Beispiele

**Allgemeine Herstellvorschrift.** In einem 1 m³-Rührreaktor mit wurden ca. 500 I Wasser vorgelegt und mit 50 kg - bezogen auf Aktivsubstanz - Emulgator vermischt. Die Lösung wurde auf 80°C erwärmt und dann die Wachskörper kontinuierlich eingerührt. Nach einer Homogenisierzeit von 30 min wurden die heißen Dispersionen in eine Sprühvorrichtung gepumpt und auf die Oberfläche einer in einem zweiten Reaktor befindlichen, auf 20°C temperierten Tensidlösung gesprüht. Die Zusammensetzung der Mischungen sowie die experimentellen Daten sind in Tabelle 1 zusammengefaßt:

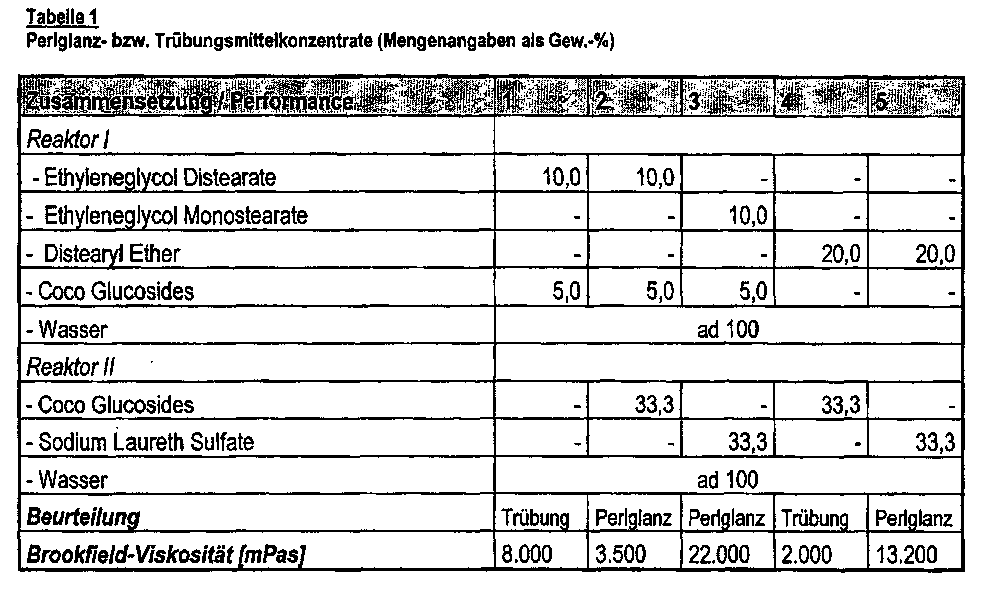

## Patentansprüche

1. Verfahren zur Herstellung von fließfähigen Perlglanz- und Trübungsmittelkonzentraten, bei dem man in einem ersten Reaktor einen wäßrigen Wachskörperpremix herstellt und diesen in einen zweiten mit Wasser gefüllten Reaktor versprüht.

2. Verfahren nach Anspruch 1, **dadurch gekennzeichnet, daß** man einen wäßrigen Premix von Wachskörpern herstellt, die ausgewählt sind aus der Gruppe, die gebildet wird von
(a1) Alkylenglycolestern,
(a2) Fettsäurealkanolamiden,
(a3) Partialglyceriden,
(a4) Estern von mehrwertigen, gegebenenfalls hydroxysubstituierten Carbonsäuren mit Fettalkoholen mit 6 bis 22 Kohlenstoffatomen,
(a5) Fettalkoholen, Fettketonen, Fettaldehyden, Fettethem und/oder Fettcarbonaten, die in Summe mindestens 24 Kohlenstoffatome aufweisen,
(a6) gegebenenfalls hydroxyfunktionalisierte Fettsäuren mit 18 bis 24 Kohlenstoffatomen,
(a7) Alkandiole mit 10 bis 18 Kohlenstoffatomen,
(a8) Ringöffnungsprodukten von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen.

3. Verfahren nach den Ansprüchen 1 und 2, **dadurch gekennzeichnet, daß** man die Wachskörper zusammen mit anionischen, nichtionischen, kationischen, amphoteren und/oder zwitterionischen Emulgatoren zu einem wäßrigen Premix verarbeitet.

4. Verfahren nach den Ansprüchen 1 bis 3, **dadurch gekennzeichnet, daß** man den wäßrigen Premix bei Temperaturen im Bereich von 60 bis 100°C herstellt.

5. Verfahren nach den Ansprüchen 1 bis 4, **dadurch gekennzeichnet, daß** man den wäßrigen Premix in eine wäßrige Lösung anionischer, nichtionischer, kationischer, amphoterer und/oder zwitterionischer Tenside versprüht.

6. Verfahren nach den Ansprüchen 1 bis 5, **dadurch gekennzeichnet, daß** man den wäßrigen Premix in Wasser oder eine wäßrige Tensidlösung versprüht, welche eine Temperatur im Bereich von 10 bis 30°C aufweist.

## Claims

1. Process for preparing flowable pearlescent and opacifier concentrates by preparing an aqueous waxy substance premix in a first reactor and spraying it into a second reactor filled with water.

2. Process according to Claim 1, **characterized in that** an aqueous premix is prepared from waxy substances which are selected from the group consisting of
(a1) alkylene glycol esters,
(a2) fatty acid alkanolamides,
(a3) partial glycerides,
(a4) esters of polybasic, optionally hydroxyl-substituted, carboxylic acids with fatty alcohols having 6 to 22 carbon atoms,
(a5) fatty alcohols, fatty ketones, fatty aldehydes, fatty ethers and/or fatty carbonates which have a total of at least 24 carbon atoms,
(a6) optionally hydroxyl-functionalized fatty acids having 18 to 24 carbon atoms,
(a7) alkanediols having 10 to 18 carbon atoms,
(a8) ring-opening products of olefin epoxides having 12 to 22 carbon atoms with fatty alcohols having 12 to 22 carbon atoms and/or polyols having 2 to 15 carbon atoms and 2 to 10 hydroxyl groups.

3. Process according to Claims 1 and 2, **characterized in that** the waxy substances are processed together with anionic, nonionic, cationic, amphoteric and/or zwitterionic emulsifiers to give an aqueous premix.

4. Process according to Claims 1 to 3, **characterized in that** the aqueous premix is prepared at temperatures in the range from 60 to 100°C.

5. Process according to Claims 1 to 4, **characterized in that** the aqueous premix is sprayed into an aqueous solution of anionic, nonionic, cationic, amphoteric and/or zwitterionic surfactants.

6. Process according to Claims 1 to 5, **characterized in that** the aqueous premix is sprayed into water or an aqueous surfactant solution which has a temperature in the range from 10 to 30°C.

## Revendications

1. Procédé de préparation de concentrés d'agents à éclat nacré d'opalescence aptes à l'écoulement, selon lequel on prépare dans un premier réacteur un prémix aqueux de solide de cire et on pulvérise celui-ci dans un deuxième réacteur rempli d'eau.

2. Procédé selon la revendication 1,
**caractérisé en ce qu'**
on prépare un prémix aqueux de solides de cire, qui sont choisis dans le groupe formé :
(a1) des esters d'alkylèneglycol,
(a2) des alkanolamides d'acide gras,
(a3) des glycérides partiels
(a4) des esters d'acide carboxylique plurifonctionnel éventuellement substitués par un hydroxy avec des alcools gras ayant de 6 à 22 atomes de carbone,
(a5) des alcools gras, des cétones grasses, des aldéhydes gras, des éthers gras, et/ou des carbonates gras qui possèdent globalement au moins 24 atomes de carbone,
(a6) éventuellement des acides gras fonctionnalisés par un hydroxy ayant de 18 à 24 atomes de carbone,
(a7) des alkanediols ayant de 10 à 18 atomes de carbone,
(a8) des produits d'ouverture de cycle d'époxydes d'oléfine ayant de 12 à 22 atomes de carbone avec des alcools gras ayant de 12 à 22 atomes de carbone et/ou des polyols ayant de 2 à 15 atomes de carbone et de 2 à 10 groupes hydroxyle.

3. Procédé selon la revendication 1 ou 2,
**caractérisé en ce qu'**
on transforme les solides de cire conjointement avec des agents émulsionnants anioniques, non ioniques, cationiques, amphotères, et/ou zwitterioniques, en un prémix aqueux.

4. Procédé selon les revendications 1 à 3,
**caractérisé en ce qu'**
on prépare le prémix aqueux à des températures dans la zone de 60 à 100°C.

5. Procédé selon les revendications 1 à 4,
**caractérisé en ce qu'**
on pulvérise le prémix aqueux dans une solution aqueuse d'agent tensioactif anionique, non ionique, cationique, amphotère et/ou zwitterionique.

6. Procédé selon les revendications 1 à 5,
**caractérisé en ce qu'**
on pulvérise le prémix aqueux dans l'eau ou dans une solution aqueuse d'agent tensioactif qui possède une température dans la zone de 10 à 30°C.
